Europäisches Patentamt

European Patent Office

Office européen des brevets

(11) Publication number: **0 354 923 B1**

## EUROPEAN PATENT SPECIFICATION

(45) Date of publication of patent specification: **29.06.94**  (51) Int. Cl.5: **A61K 49/02**, C07B 59/00

(21) Application number: **88904758.5**

(22) Date of filing: **01.04.88**

(86) International application number:
**PCT/US88/01048**

(87) International publication number:
**WO 88/07382 (06.10.88 88/22)**

(54) **METHOD FOR LABELLING ANTIBODIES WITH A METAL ION.**

(30) Priority: **02.04.87 US 34003**
**03.12.87 US 128328**

(43) Date of publication of application:
**21.02.90 Bulletin  90/08**

(45) Publication of the grant of the patent:
**29.06.94 Bulletin  94/26**

(84) Designated Contracting States:
**AT BE CH DE FR GB IT LI LU NL SE**

(56) References cited:
EP-A- 0 135 160     EP-A- 0 188 256
EP-A- 0 237 150     FR-A- 2 269 354
US-A- 4 027 005     US-A- 4 418 052
US-A- 4 652 440

The International Journal of Radiation and
Isotopes, volume 36, no. 6, June 1985, Per-
gamon Press Ltd, (Oxford, GB), L.L.-Y.
Hwang et al. : "Complexes of technetium
with polyhydric liggands "

(73) Proprietor: **CENTOCOR, INC.**

244 Great Valley Parkway
Malvern, PA 19355(US)

(72) Inventor: **PAK, Koon, Yan**
1010 Cooper Avenue
Norristown, PA 19401(US)
Inventor: **DEAN, Richard., T.**
Three Heron Hill Drive
Downingtown, PA 19335(US)
Inventor: **MATTIS, Jeffrey, A.**
1212 Upton Circle
West Chester, PA 19380(US)
Inventor: **BUTTRAM, Scott**
329 Horseshoe Lane
Downingtown, PA 19335(US)
Inventor: **LISTER-JAMES, John**
Ten Stonehedge Drive
Glenmoore, PA 19343(US)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(74) Representative: **Holdcroft, James Gerald, Dr. et al**
**Graham Watt & Co.,**
**Riverhead**
**Sevenoaks, Kent TN13 2BN (GB)**

**Description**

Background of the Invention

Proteins have been labeled with various radiometals and other radioisotopic elements for use in immunodiagnostic and immunotherapeutic procedures. Some radiometals have superior properties for use in these techniques. Technetium-99m is an ideal radionuclide for scintigraphic imaging because of its nuclear properties. It has a single photon energy of 140keV, a half-life of about 6 hours, and it is readily available from a $^{99}$Mo-$^{99m}$Tc generator. Rhenium radioisotopes are beta-emitters which can kill target cells and thus are useful in therapy. Rhenium-186 and -188 also have gamma emission which, as an added feature, allows it to be imaged by scintigraphic techniques.

Two general approaches have been taken to label proteins such as antibodies with radiometals. The first is the direct labeling method by which the radiometal is bound to the protein molecule itself. The second is the indirect labeling method in which a chelating agent is coupled to the protein and the radiometal is attached to the protein via the chelating agent.

Rhodes discloses a method of direct labeling of protein with technetium-99m which involves ligand solid phase exchange. See U.S. Patent 4,305,922. According to the method of Rhodes, pertechnetate is reduced to technetium IV and then applied onto a Sephadex$^R$ column. The reduced technetium-99m binds to the Sephadex$^R$ material. A solution of the protein to be labeled is poured onto the top of the Sephadex column where it is allowed to remain so that ligand exchange occurs. As a result, the technetium-99m is transferred preferentially from the Sephadex material to the protein. The protein may be pretreated with a stannous chloride (a procedure called "pretinning") to enhance transfer of the radiometal to the protein. See U.S. Patent No. 4,424,200.

Various attempts have been made to label proteins with radiometals by the indirect approach. In one such approach, a chelating agent such a diethylenetriaminepentaacetic acid (DTPA) is conjugated onto the protein and then the metal ion is labeled onto the chelating agent attached to the protein molecule. For example, Khaw et al., Science 209: 295-297 (1980) discloses antibodies to cardiac myosin labeled with indium-111 via DTPA and use of the labeled antibodies to image for myocardial infarction. See also, Krejcarek et al., Biochem. Biophys. Res. Commun. 77: 581-585 (1977); Childs, R.L. and Hnatowich, D.J., J. Nucl. Med. 26: 293 (1985); U.S. patent 4,652,440 Paik et al., (1987). In a more recent approach, Fritzberg et al. describe the use particular diaminodithiol and diamidodithiol groups, as a chelating agents. Fritzberg et al, J. Nucl. Med. 27:957 (1986); European Patent Application 86100360.6. In U.S. patent 4,027,005, Adler et al. discloses technetium-labeled polyhydroxycarboxylic acids for use as radionuclide diagnostic agents.

Various degrees of success have been achieved with both the direct and indirect methods of labeling proteins with radiometals. However, the labeled product is often unstable in vivo. Further, techniques for purifying the labeled product before use are often required. A need exists for improved methods for stably labeling proteins for radioimmunodiagnostic and radioimmunotherapeutic procedures.

This invention pertains to a simple, rapid and efficient method of labeling sulfhydryl-containing antibodies or antibody fragments with the radiometals technetium-99m rhenium-186, rhenium-188, rhenium-189 and rhenium-191. In general, the method comprises:

    a. forming an aqueouos mixture of
        (i) the radiometal in an oxidized form; and
        (ii) a reducing agent and a water-soluble polyhydroxycarboxylic ligand selected from saccharic acid, galactaric acid, arabonic acid and salts thereof which is capable of forming a stable complex with the radiometal in its reduced state and quantitatively exchanging the radiometal with a sulfhydryl-containing antibody; and
    b. contacting the mixture with a sulfhydryl-containing antibody or antibody fragment to produce a radiometal-labeled antibody or antibody fragment.

EP-A-0 237 150 was published on 16.09.87 and claims a priority date of 12.03.86, and therefore is acknowledged pursuant to Art. 54(3) EPC. It discloses a method and kit for preparing a radionuclide-labeled protein from a protein precursor having at least one di-sulfide linkage. The method involves the use of a disulfide reducing agent, and the labeling species is e.g. a labile, soluble complex of reduced Tc-99m, complexed by use of a weak chelating agent. Specific examples of the chelating agents disclosed in EP-A-0 237 150 are gluconate, glucoheptonate, tartrate and malonate species. There is no teaching of the ligands saccharic acid, galactaric acid, arabonic acid and salts thereof, the use of which the present invention claims.

The technetium-99m labeled antibodies or antibody fragments of the present invention are useful for radioimminodiagnostic purposes such as immunoscintigraphy. The rhenium labeled antibodies or antibody fragments can be used for therapy.

The especially preferred ligand is saccharic acid, (D-glucaric acid). Saccharic acid quickly and stably complexes with technetium-99m in its reduced state and without the formation of significant technetium colloids. When contacted with a sulfhydryl-containing antibody, Technetium-99mm is preferentially transferred to the antibody to form a stable labeled antibody.

The preferred reducing agents for use in the method are stannous reducing agents such as stannous chloride. These reagents effectively reduce technetium and are pharmacologically acceptable.

The method of this invention can be used to label whole antibodies (e.g., IgG) or antibody fragments (e.g., Fab'). Whole antibodies can be reduced with the reducing agent dithiothreitol (DTT) for example, to produce sulfhydryl containing antibodies. Fab' fragments are especially suited for labeling by the procedure. Under nonoxidizing conditions, these fragments contain free sulfhydryl groups (as they are produced by reduction of disulfide bridges present in $F(ab)'_2$ fragments. For most radioimmunodiagnostic techniques, antibody fragments such as Fab' fragments are preferred and thus, the labeling procedure of this invention is particularly suited for preparing radiopharmaceuticals for these techniques.

The method of radiolabeling antibody or antibody fragments with the designated radiometals can be performed as a simple two-vial procedure. For this purpose, kits can be provided with the reagents in a form ready for use on site by the clinician. For example, such a kit can include a first vial containing a reducing agent (e.g. stannous ions) and the water soluble ligand (e.g. saccharic acid or a salt thereof) and a second vial containing a Fab' fragment suited for the particular diagnostic or therapeutic procedure. The reactions are preferably carried out in an aqueous medium although the reagents may be supplied in lyophilized, frozen or aqueous form. For the preparation of technetium-99m labeled fragments, technetium-99m (generally in the form of pertechnetate) is added to the first vial and then the contents of the first and second vial are mixed and incubated for a time sufficient to effect a quantitative transfer of the technetium-99m to the Fab' fragment. The composition can then be injected into the patient without purification. For radiolabeling with rhenium, rhenium isotopes (in the form of a perrhenate) are used in place of the technetium. The rhenium labeled Fab' fragment is also suitable for injection without purification.

In a preferred embodiment, the radiolabeling can be performed in a single vial. A kit can include a single vial containing an antibody mixture comprised of a sulfhydryl containing antibody or antibody fragment, a reducing agent (e.g. stannous ions), and preferably a water soluble ligand (e.g. D-glucaric acid or a salt thereof). The antibody mixture is preferably supplied in lyophilized form although frozen or aqueous forms are also suitable. Technetium-99m (generally in the form of $^{99m}Tc$ pertechnetate) is added to the vial and the resulting mixture is incubated for a time sufficient to effect a quantitative transfer of the technetium-99m to the antibody or antibody fragment. This composition can then be injected into the patient without purification. The technetium-99m-labeled antibodies and antibody fragments prepared by the method of this invention can be used for diagnostic purposes such as immunoscintigraphy of tumor, myocardial infarction, thromboses or bacterial abscess. Rhenium-labeled antibodies can be used to selectively deliver a rhenium radioisotope in vivo for therapy.

The method of this invention has several important advantages. As mentioned, the ligands employed are capable of complexing technetium-99m quantitatively in stable form as a complex without the formation of a significant amount of technetium colloid. Upon contact with a sulfhydryl containing antibody under appropriate conditions, the complexed technetium-99m is transferred substantially quantitatively to sulfhydryl-containing antibodies so that radiodiagnostic composition can be prepared with very high specific activity. The antibody or antibody fragments labeled by the method retain their original immunoreactivity and consequently their target specificity. The radiolabeled antibody is stable in solution and in serum. When Fab' fragments labeled by the method are administered in vivo very little label accumulates in the liver which indicates that the labeled antibody is stable in vivo. In addition, the labeling method can be performed rapidly (it can be completed in less than one hour) and the method can be performed at room temperature and at pH 5-9. The labeled product does not require purification before use.

Brief Description of the Figures

Figure 1 shows gamma scintigrams of a dog at various times after injection of technetium-99m labeled myosin-specific Fab' fragment.

Figure 2 shows gamma scintigrams of the same dog taken from different views.

Figure 3 shows the results of mouse biodistribution studies in blood conducted with antifibrin T2G1S prepared in single vial and double vial labeling kits.

Figure 4 shows the results of mouse biodistribution studies in liver conducted with antifibrin T2G1S prepared in single vial and double vial labeling kits.

Figure 5 shows the results of mouse biodistribution studies in spleen conducted with antifibrin T2G1S prepared in single vial and double vial labeling kits.

Figure 6 shows the results of mouse biodistribution studies in kidney conducted with antifibrin T2G1S prepared in single vial and double vial labeling kits.

Figure 7 shows the results of mouse biodistribution studies in large intestine conducted with antifibrin T2G1S prepared in single vial and double vial labeling kits.

Detailed Description of the Invention

In one embodiment, the method of this invention is performed by reacting Technetium-99m (in an oxidized state) with a water-soluble ligand in the presence of a reducing agent to form a stable complex between technetium-99m in a reduced state (e.g., IV or V valence state) and the ligand and then reacting the complex with an antibody or antibody fragment which contains one or more sulfhydryl groups. In the preferred embodiment for labeling a sulfhydryl-containing antibody with technetium-99m, aqueous sodium 99m-pertechnetate is mixed with a aqueous solution of a stannous reducing agent and saccharic acid (or a salt thereof) to form a $^{99m}Tc$-saccharate complex. The complex is then contacted with an Fab' fragment and incubated for a period of time and under conditions which allow an exchange of technetium-99m from the complex to the Fab' fragment to form a technetium-labeled Fab' fragment. The entire procedure can be conducted in less than one hour at room temperature and at a pH of about 5-9. Under these conditions an essentially complete transfer of technetium-99m (from the 99m-Tc-saccharate complex to the antibody protein) can be attained without significant loss of antibody immunoreactivity.

Single vial technique

This invention provides a method of radiolabeling protein in a single vial. The reduction of the oxidized form of technetium-99m and the radiolabeling reaction (i.e., the coupling of the radioisotope to protein) are achieved in the same vial. As used herein, the term "vial" refers to any type of reaction vessel and is not intended to be limiting in any way. The method is simple, efficient, and reproducible and it minimizes the safety hazards to persons performing the radiolabeling. The method of this invention is particularly suited for labeling antibodies (polyclonal and monoclonal) for diagnosis. Antibodies can be labeled by this method to a high specific activity with minimal loss of immunoreactivity.

Advantages with the present one vial method over methods using two vials and other known methods for labeling with technetium-99m include: (1) Rapid labeling at ambient conditions. Labeling yields greater than 90% can be achieved in 5-15 minutes at ambient temperature without heating. The clinical advantages of near instantaneous preparation of a diagnostic agent can be substantial. (2) Stability of the lyophilized formulation of the single vial method is superior to the comparable formulation employed in a two vial method. (3) Biodistribution studies of the product resulting from the one vial method show statistically significant differences in key major organs. Uptake in kidney and liver is lower with product produced by this method. Blood clearance is significantly faster. These types of differences would indicate that product from this method would produce lower background, lower absorbed dose to critical organs and faster blood clearance resulting in faster ability to image areas of interest. All these would be substantial clinical advantages. (4) Plasma stability of the product is greater. This provides more viable intact product to serve as the diagnostic agent in vivo.

In a preferred embodiment, an antibody mixture comprised sylfhydryl containing antibody or antibody fragment, a reducing agent and a water soluble ligand are added to a vial. Preferably, sealable reaction vial is used which has means for the introduction and withdrawal of reagent preferably under sterile or semi-sterile conditions. A vial which contains a port for syringe injection is preferred. All reagents can be injected and withdrawn from the reaction vial by syringe, thereby reducing the risk of exposure to radio- or biohazardous reagents. In a most preferred embodiment, the mixture is lyophilized and the vial is presealed and supplied for use in that form. In order to label the antibody or antibody fragment, technetium-99m in an oxidized state is contacted with the antibody mixture. The radiolabelling reaction is then allowed to proceed. The duration and condition of incubation are not critical. Preferably, incubation is conducted for a period from about one minute to about 60 minutes, and most preferably from about 5 minutes to about 30 minutes.

After completion of the labeling reaction, the labeled antibody or antibody fragment is withdrawn from the vial. No separation or purification is required. The entire procedure can be conducted in less than 15 minutes at ambient temperature and at a pH of about 5-9. Under these conditions an essentially complete labeling of the antibody or antibody fragment with technetium-99m can be attained without significant loss of antibody immunoreactivity.

The various reagents used in the method and the parameters of the method are discussed in detail below.

## The Ligands

In general, the ligands useful in the method of this invention are water-soluble (or can be made water soluble) chelators which are capable of complexing technetium-99m or any of the rhenium radioisotopes in their reduced state to form a stable metal ion/ligand complex. The complex is capable of exchanging the technetium-99 with a sulfhydryl containing antibody or antibody fragment.

The ligands used in this invention are polyhydroxydicarboxylic acids having a molecular weight of less than about 10,000 daltons, and are selected from saccharic acid, galactaric acid, arabonic acid, and salts thereof.

The particularly preferred ligand for use in this method is saccharic acid. As mentioned, saccharic acid complexes with technetium-99m quickly to form a stable technetium-99m-saccharate complex. Upon contact with a sulfhydryl-containing antibody or antibody fragment, substantially quantitative transfer of technetium-99m from the complex to the protein is achieved rapidly and under mild conditions. As described below, it is believed that the technetium-99m is preferentially transfered to favored binding sites on the protein molecules. This preferential transfer results in a labeled antibody or fragment which is immunoreactive and exceptionally stable in vivo.

## Reducing Agents

Reducing agents for use in the method are physiologically acceptable for reducing technetium-99m from its oxidized state to the IV or IV oxidization state or for reducing rhenium from its oxidized state. Examples of reducing agents which can be used in the method are stannous chloride, stannous fluoride, stannous tartarate, and sodium dithionite; the preferred agents are stannous reducing agents especially stannous chloride.

## Radioisotopes of technetium and rhenium

The source of Technetium-99m should preferably be water soluble. Preferred sources are alkali and alkaline earth metal pertechnetate ($TcO_4^-$). The technetium-99m is most preferably obtained in the form of fresh sodium pertechnetate from a sterile technetium-99m generator (e.g., from a conventional 99Mo/99mTc generator). Any other source of physiologically acceptable technetium-99m, however, may be used.

Rhenium radioisotopes (the isotopes 186, 188, 189 and 191) in the form of perrhenate salts can be produced by suitable reactor technology or made by a suitable generator. The perrhenate salts are stable, soluble salts and behave similarly to pertechnetate. Perrhenate requires a slightly greater reduction potential to reduce, and tends to return to perrhenate in the presence of oxygen more readily than pertechnetate. For this reason, different conditions may be required to reduce and stabilize rhenium in its reduced state. These can be ascertained empirically by a person of ordinary skill in the art.

## Sulfhydryl-containing antibodies or antibody fragments

The sulfhydryl containing whole antibodies or lower molecular weight antibody fragments can be labeled by the method of this invention. It is believed that sulfhydryl groups constitute at least a part of favored binding sites which exist on molecules and that by the method of this invention, the radiometals are preferentially exchanged from the radiometal-ligand complex to these favored sites on the molecules. The preferential labeling of these sites on the antibodies molecules results in labeled antibodies of exceptional stability.

Whole antibodies (e.g. IgG) can be provided with sulfhydryl groups by reducing the antibodies with a reducing agent such as dithiothreitol DTT. Treatment with DTT exposes the sulfhydryl groups by reducing disulfide bridges.

For most immunodiagnostic procedures, antibody fragments are preferred reagents. Antibody fragments have a number of advantages over whole antibodies for imaging procedures including, in general, more rapid distribution and accumulation at target site and less immunogenicity. Fab' fragments are monovalent antibody binding which contain free sulfhydryl groups (when maintained under non-oxidizing conditions). These fragments can be labelled efficiently by the method of this invention.

6

Fab' fragments can be prepared from whole antibodies as follows: An antibody molecule is first treated with an endopeptidase such as pepsin to remove the Fc portion of the antibody molecule. The resultant F-(ab)'$_2$ fragment is treated with a reducing agent such as DTT or cysteine to break disulfide bonds present on the F(ab)'$_2$ fragment resulting in exposed the sulfhydryl groups present on the molecules and thereby producing two Fab' molecules for each antibody molecule.

Reaction Conditions

The amount of reducing agent is the amount necessary to reduce the technetium to provide for the binding to the ligand in a reduced state. In a preferred mode, stannous chloride (SnCl$_2$) is the reducing agent and can range from 1-1,000 ug/ml preferably about 30-500 ug/ml. The amount of saccharic acid (as potassium saccharate) can range from about 0.5 mg/ml up to the amount maximally soluble in the medium. Preferred amounts of saccharic acid range from 30-15 ug/ml. The amount of antibody (or fragment) can range from 0.01 to about 30 mg/ml preverably about .17 to about 1.5 mg/ml. Finally, technetium-99m in the form of pertechnetate can be in amounts used up to about 500 uCi/ml preferably about 1-50 mCi/ml. The amount of mCi per mg of antibody is preferably about 3-150.

The reaction between the and the metal ion-transfer ligand complex is preferably carried out in an aqueous solution at a pH at which the protein is stable. By "stable", it is meant that the protein remains soluble and retains its biological activity. Normally, the pH for the reaction will be a pH from about 5 to 9, the preferred pH being about 6-8. The metal ion-transfer chelate complex and the antibody are incubated, preferably at a temperature from about 20°C to about 60°C, most preferably from about 20°C to about 37°C, for a sufficient amount of time to allow transfer of the metal ion from the ligand complex to the antibody. Generally, less than one hour is sufficient to complete the transfer reaction under these conditions.

Kits For Performing The Method

The reagent for performing the labeling method can be assembled in kits for convenient performance of the method in the clinic. At minimum, a kit for radiolabeling antibody or antibody fragments with the radiometals can consist of a one component a vial (sealed and sterile) containing a reducing agent (preferably stannous ions) and saccharic acid or a salt thereof. These kits can be used when the antibody or antibody fragment is provided by the user.

Kits may also include a second vial containing the sulfhydryl-containing antibody or antibody fragment to be labeled. Two component kits would include:

a. a vial containing a reducing agent and a water-soluble transfer ligand; and

b. a sulfhydryl-containing antibody or antibody fragment under non-oxiding conditions.

Kits can be designed to contain the appropriate antibody or antibody fragment(s) for any particular immunodiagnostic or immunotherapeutic procedure (some of which are discussed below).

The reagents in the kit can be provided in aqueous, lyophilized or from form. Lyophilized preparations can be diluted with aqueous medium upon use. The amount of reagents in each vial can vary according to the chosen parameters of the method (see above under Reaction Conditions).

When reagents are provided as a two component kit, as described, the labeling procedure can be performed simply as a two vial technique. Technetium-99m (for example, in the form of aqueous sodium pertechnetate) is added to the vial containing the reducing agent and the water-soluble ligand in aqueous solution. The contents of the two vials are then mixed and incubated for a time sufficient to effect labeling of the antibody or antibody fragment. The radiolabeled antibody or antibody fragment can then be used immediately without purification.

One vial kits for performing the method

The reagents for performing the present labeling method are assemlbled in single vial kit for convenient performance in the clinic. In one embodiment, the kit contains one vial (sealed and sterile) containing a sulfhydryl containing antibody or antibody fragment, a reducing agent (preferably stannous ions) and a water soluble ligand (preferably D-glucaric acid or a salt thereof). These kits can be used when technetium-99m is provided by the user. The kits are designed to contain the appropriate antibody or antibody fragment(s) for any particular immunodiagnostic or immunotherapeutic procedure (some of which are discussed below).

The reagents in the kit can be provided in aqueous, frozen or lyophilized form. Lyophilized preparations can be diluted with aqueous medium upon use. The amount of reagents in each vial can vary according to the chosen parameters of the method (see above under Reaction Conditions). The labeling procedure can be performed simply by adding technetium-99m (for example, in the form of aqueous sodium pertechnetate) to the vial containing the antibody or antibody fragment, reducing agent and, in a preferred embodiment, water soluble ligand. The contents of the vial are then mixed and incubated for a time sufficient to effect labeling of the antibody or antibody fragment. The radiolabeled antibody or antibody fragment can then be used immediately without purification.

Use of the Labeled Antibodies in Immunodiagnostics

Technetium-99m labeled antibodies or antibody fragments can be used in immunoscintigraphy. One important use is in the imaging of tumors. As mentioned, antibody fragments are preferred for most immunoscintigraphic techniques. Labeled Fab' fragments of tumor specific antibodies can be prepared and used to image primary or secondary tumors. In general, the technetium-99m labeled antibody fragment is prepared by forming an aqueous mixture of (i) 99m Tc; and (ii) a reducing agent and a water-soluble ligand; and contacting the mixture with an Fab' fragment specific for the tumor.

The labeled Fab' fragment can then be injected parenterally (preferably intraveneously) into a subject. After injection, sufficient time is allowed for the labeled Fab' fragment to accumulate at the site of the tumor. The subject is then scanned with a gamma camera to detect the gamma emission of the technetium-99m and to thereby to obtain an image of the tumor. In this way the tumor can be localized and its size can be determined.

Tumor-specific antibody fragments for use in these procedures can be derived from anticolorectal cancer antibody, antilung cancer antibody anti-ovarian cancer antibody, antibreast cancer antibody, and antiprostate cancer antibody. Some specific examples of tumor specific antibodies which can be labeled by the method of this invention and used to image tumors are the monoclonal antibodies 17-1A and 19-9 (gastrointestinal), CA 125 (ovarian) and 103D2 (breast).

Antibodies labeled by the method of this invention can be used to label myocardial infarcts. The imaging of myocardial infarcts to determine their size and location is described by Haber, U.S. Patent No. 4,421,735. In brief, employing the labelling method of this invention, an image of a mycocardial infarct in a subject can be obtained by first preparing a Tc-99m labeled myosin specific Fab' fragment by first forming an aqueous mixture of (i) $^{99m}$Tc and (ii) a reducing agent and a water soluble ligand for $^{99m}$Tc; and then contacting the mixture with a myosin specific Fab' fragment. The labeled myosin specific fragment is then intraveneously injected into a subject (for example, after coronary occlusion). The labeled fragment is allowed to localize at the site of the infarct and an image of the infarct is obtained by scanning the area of the heart with a gamma camera.

A preferred antibody for production of labeled myosin-specific Fab' fragments is the monoclonal antibody R11D10.

In addition, fibrin-specific Fab' fragments can be labelled by the procedure of this invention to provide reagents for imaging blood clots. A Tc-99m labeled fibrin-specific fragment is prepared by forming an aqueous mixture of (i) $^{99m}$Tc and (ii) a reducing agent and a water soluble ligand for $^{99m}$Tc and contacting the mixture with a fibrin specific Fab' fragment. The $^{99m}$Tc-labeled fibrin specific fragment is injected into the subject. After allowing the fragment to localize at the site of the blood clot, the subject is scanned to obtain an image of the clot. Fibrin-specific antibodies which are not cross-reactive with fibrinogen are the preferred antibodies for this imaging technique.

Antibody fragments specific for bacteria can be used in immunoscintigraphic techniques for obtaining an image of a bacterial abscess in a subject. For this purpose, anti-bacterial or anti-macrophage antibody fragments are employed. Antibodies against a common determinant of gram-negative bacteria (e.g., anti-lipid A antibody) can be used to image an abscess caused by a gram-negative microorganism. The antibody is labeled with technetium-99m as described above injected into the subject and allowed to localize at the abscess. The subject is then scanned with the photoscanning equipment to obtain an image of the abscess.

Radioimmunotherapeutics

Rhenium-labeled antibody or antibody fragments can be used to selectively deliver rhenium radioisotopes to target cells in vivo. For example, rhenium labeled antibodies can selectively seek out and destroy cancer cells. For this purpose, tumor specific antibodies, such as those described above, can be labeled by the method of this invention and the resulting labeled antibody can be injected parenterally into a subjected afflicted with the tumor.

The invention is further illustration by the following exemplification.

Exemplification

EXAMPLE 1

Radiolabeling of Antimyosin Antibody R11D10 Fab' with Technetium-99m using $^{99m}Tc^-$ Saccharate

Preparation of $^{99m}Tc$-Saccharate

Monopotassium saccharate (25 mg) was dissolved in 0.2M bicarbonate (1.0 ml) at pH 8.0. To 500 ul of saccharate solution was added 40 ul of stannous chloride (2.5 mg/ml) in 0.1M acetic acid followed by 500 ul of Tc-99m generator eluate (__60mCi/mg protein). The resulting solution was allowed to stand for 5 minutes at room temperature and then analyzed for radiochemical purity by paper chromatography (Whatman 3MM, 60% $CH_3CN$:40%$H_2O$).

Preparation of R11D10 Fab'

Antimyosin monoclonal antibody R11D10 $F(ab')_2$ 5mg/ml in 40mM TRIS pH 7.0 was reduced with 10mM DTT for 60 minutes at room temperature and then passed through a Sephadex G-25 column to remove the reducing agent. The resulting solution contained __80% Fab' fragment by gel-filtration HPLC.

Labeling of R11D10 Fab' using $^{99m}Tc$-Saccharate

Antimyosin antibody R11D10 Fab' (500 ul of a 1 mg/ml solution) in 50mM phosphate, 0.35 mM $ZnCl_2$, pH 6.5 was mixed with 500 ul of $^{99m}Tc$-saccharate solution and allowed to stand at room temperature for 5-60 minutes. The resulting $^{99m}Tc$-labeled protein was analyzed for radiochemical purity by paper chromatography (Whatman 3MM; 60% $CH_3CN$: 40%$H_2O$) and gel-filtration HPLC, and for immunoreactivity using a myosin affinity column.

EXAMPLE 2

The effect of Saccharate Concentration on the Formation of $^{99m}Tc$-Saccharate

$^{99m}Tc$-Saccharate was prepared as described in Example 1 using different concentrations of potassium saccharate (0.09-12.25 mg/ml). The products were analyzed by paper chromatography (Whatman 3MM, 60% $CH_3CN$/40% $H_2O$; $^{99m}TcO_4^-$ Rf = 1.0, $^{99m}Tc$-saccharate, Rf = 0.4; $^{99m}TcO_2.x H_2O$, Rf = 0). The data in Table I show that a concentration of 6 mg/ml potassium saccharate in 0.2M bicarbonate is sufficient to completely stabilize the reduced technetium.

EXAMPLE 3

Stability of $^{99m}Tc$-Saccharate

Samples of $^{99m}Tc$-saccharate prepared from 6 and 12 mg/ml potassium saccharate were analyzed over a period of 7 hours. The results (Table II) indicated that the preparation from 12 mg/ml saccharate was more stable and was stable for a period of about 2 hours.

EXAMPLE 4

The effect of Antibody Concentration on the Labeling of R11D10 Fab' using $^{99m}Tc$-Saccharate

$^{99m}Tc$-labeled R11D10 Fab' was prepared as described in Example 1 using various protein concentrations up to 1250 ug/ml. After 1 hour, the reaction mixtures were analyzed by paper chromatography and HPLC. The results (Table III) showed that the radiochemical yield was dependent upon the concentration of the protein and that quantitative labeling could be obtained in 1 hour using at least 340 ug/ml.

EXAMPLE 5

Evaluation of the Transfer of Technetium from $^{99m}$Tc-Saccharate to Non-Reduced Antibody/Fragments compared to Fab' Fragments

100 ul of whole antibody (2 mg/ml), F(ab')$_2$ (2mg/ml), Fab' (1mg/ml) of antimyosin antibody R11D10, antipancreatic antibody 19-9 and anticolorectal antibody 17-1A were incubated with 100 ul $^{99m}$Tc-saccharate solution at room temperatures for 1 and 3 hours. The resulting products were analyzed by paper chromatography. The results (Table IV) showed that the labeling of non-reduced antibody/fragments was less than 5% versus quantitative labeling of the Fab' fragments.

EXAMPLE 6

Technetium-99m Labeling of Anti-Colorectal Antibody 17-1A Fab' and Antimyosin Antibody R11D10 Fab'

Both antibody fragments were prepared and labeled as described in Example 1. Gel filtration HPLC analysis of the products after three hours at room temperature shows that for the 17-1A 35% of the protein was in the form of F(ab')$_2$ and 65% Fab' whereas for R11D10 23% was in the form of F(ab')$_2$ and 77% as Fab'. However, radioactive detection showed that 80% of the radioactivity was associated with the Fab' peak for both antibodies. These results shows that the $^{99mTc-saccharate}$ preferentially labels the Fab' fragments.

EXAMPLE 7

Radiochemical stability of $^{99m}$Tc-Labeled Fab' Antibody Fragments

$^{99m}$Tc-labeled 17-1A Fab' and R11D10 Fab' were incubated at 37°C for 1 hour in the presence and absence of human plasma. The results (Table V) showed that 80% of the technetium remained bound to the antibody for over 20 hours even in the presence of plasma.

EXAMPLE 8

Immunoreactivity of $^{99m}$Tc-labeled R11D10 Fab'

Immunoreactivity of $^{99m}$Tc-R11D10 Fab' preparation was determined using a myosin affinity column. $^{99m}$Tc-17-1A was used as a control to estimate non-specific binding. Each labeled protein was incubated at 37°C in the Presence of human plasma.

As shown in Table VI, the $^{99m}$Tc-labeled R11D10 Fab' was nearly 80% immunoreactive after 3 hours and 70% immunoreactive after 20 hours. The latter corresponded to 80% retention of immunoreactivity found immediately after labeling.

EXAMPLE 9

Detection of Myocardial Infarct In The Dog Using $^{99m}$Tc-R11D10 Fab'

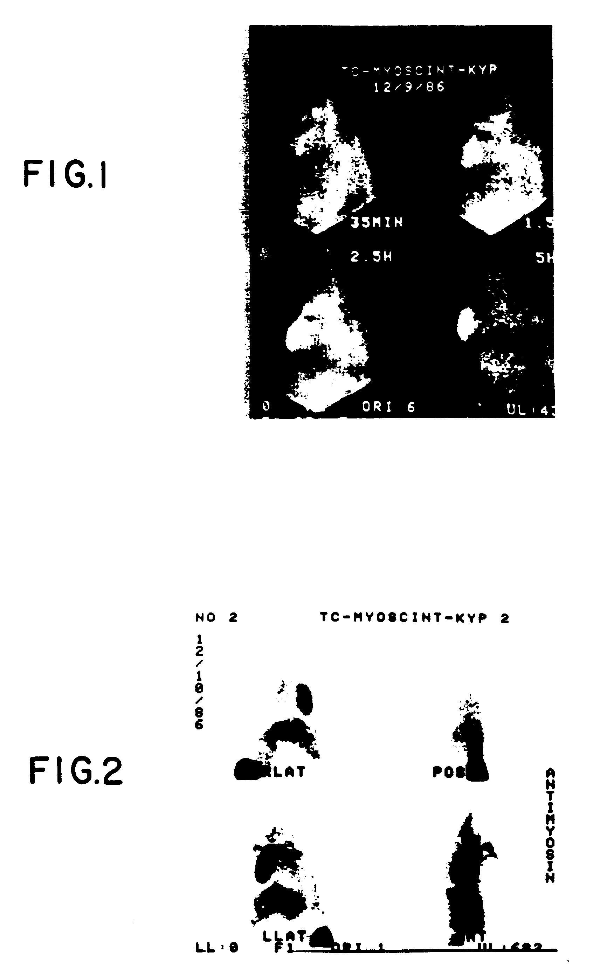

Mongrel dogs (n = 6) were anesthetized with I.V. pentobarbitol (30 mg/kg), and respiration maintained on a Harvard respirator. Left thoracotomy was performed, the heart suspended in a pericardial cradle and a segment of the left anterior descending coronary artery approximately two thirds the distance from the apex to the base was dissected free. The LAD was then occluded with a silk ligature. After three hours of LAD occlusion, the occlusive ligature was removed and reperfusion was established. At 15 minutes of reperfusion, 200 uCi of indium-111 labeled R11D10 Fab-DTPA was injected and 30 seconds later, 10mCi of technetium labeled R11D10 Fab' was injected. Serial imaging with a gamma camera was initiated immediately upon tracer administration. Figure 1 shows the gamma scintigrams of a dog after 35 min. (upper left), 1.5 hours (upper right), 2.5 hours (lower left) and 5 hours (lower right) of antibody injection. Figure 2 shows the gamma images of the same dog as shown in Figure 1, right lateral (upper left), posterior anterior (lower right) views. Clear myocardial infarct images were observed in all views except the posterior position. More importantly, this figure shows no significant liver uptake 3 hours after injection of Tc-R11D10-Fab'.

10

EXAMPLE 10

Biodistribution Studies of Technetium-99m Labeled R11D10 Fab' and of the Indium-111 Labeled R11D10 Fab-DTPA in Mice

Biodistribution studies were carried out in Balb/c mice. The mice (4 mice per group) were injected I.V. with either 150 uCi of technetium-99m labeled R11D10 Fab' (4 uCi/ug) or 10 uCi of indium-111 labeled R11D10 Fab-DTPA (4 uCi/ug).

Groups of mice were sacrificed at 1,4 and 8 hours after receiving the injections and organs removed, weighed and counted. Table VII summarizes the percent injected dose per gram obtained for each preparation.

The $^{99m}$Tc-R11D10 Fab' cleared rapidly from both the blood and liver. The percent of injected dose for Tc-R11D10 Fab' in the blood at 1 hour was 13.6% and dropped to 2.0% after eight hours. A similar drop in radioactivity was observed in the liver at the latter time point (6.4% in 1 hour and 2.4% in eight hours). However, the indium-111 labeled preparation showed much higher radioactivity in both liver (10.8%) and blood (5.1%) at the eighth hour after injection.

EXAMPLE 11

R11D10 Fab' With Technetium-99m Using $^{99m}$Tc-Arabonate

Preparation of $^{99m}$Tc-Arabonate

Monopotassium arabonate (20 mg) was dissolved in 0.1M $Na_2CO_3$ (1.0 ml) at pH 10.0. To 500 ul of arabonate solution was added 500 ul of Tc-99m generator eluate (approx. 60mCi/mg protein) followed by 40 ul of stannous chloride (2.5 mg/ml) in 0.1M acetic acid. The resulting solution was allowed to stand at room temperature for 30 minutes and then adjusted to pH 7 using 1.0M hydrochloric acid.

The sample was analyzed for radiochemical purity by paper chromatography (Whatman 3MM, 60% $CH_3CN$:40% $H_2O$).

Preparation of R11D10 Fab'

The same procedure as outlined in Example 1 was employed for preparation of R11D10 Fab'.

Preparation of R11D10 Fab' Using $^{99m}$Tc-Arabonate

Antimyosin antibody R11D10 Fab' (500 ul of a 1 mg/ml solution) in 50mM phosphate, 0.35 mM $ZnCl_2$, pH 6.5 was mixed with 500 ul of $^{99m}$Tc-arabonate solution and allowed to stand at room temperature for 60 minutes. The resulting $^{99m}$Tc-labeled protein was analyzed for radiochemical purity as previously noted in Example 1. The results showed quantitative transfer of $^{99m}$Tc to the protein under these conditions.

TABLES

Table 1

| Percent of $^{99m}TcO_2$ and $^{99m}Tc$-Saccharate After Incubation at Room Temperature for 1 Hr. at Various Concentrations of Saccharic Acid as Analyzed by Paper Chromatography. | | |
|---|---|---|
| Saccharic Acid (mg/ml) $^{99m}Tc$-Saccharate | % $^{99m}TcO2$ | % |
| 12.25 | .0 | 100.0 |
| 6.12 | .0 | 100.0 |
| 3.06 | 11.5 | 88.5 |
| 1.53 | 19.5 | 80.5 |
| 0.76 | 24.4 | 75.6 |
| 0.38 | 30.0 | 70.0 |
| 0.19 | 41.0 | 59.0 |
| 0.09 | 57.0 | 43.0 |

TableII

| Stability of $^{99m}Tc$-Saccharate at room temperature | | | | |
|---|---|---|---|---|
| Time Hours | 6.12 mg/ml | | 12.24 mg/ml | |
| | % Tc-SACC | %Tc04$^-$ | % Tc-SACC | %Tc04$^-$ |
| 1 | 95 | 5 | 95 | 5 |
| 3 | 76 | 24 | 82 | 18 |
| 5 | 45 | 55 | 62 | 38 |
| 7 | 36 | 64 | 60 | 40 |

Table III

| Percent of $^{99m}Tc$-Labeled R11D10 Fab' After Labeling with $^{99m}Tc$-Saccharate at Different Protein Concentration as Analyzed by Paper and HPLC Gel Filtration Chromatography. | | |
|---|---|---|
| Protein Concentration (ug/ml) | % $^{99m}Tc$-Labeled Ab | % $^{99m}Tc$-Saccharate |
| 1250 | 100.0 | 0 |
| 340 | 100.0 | 0 |
| 165 | 72.0 | 28.0 |
| 133 | 66.2 | 33.8 |
| 100 | 67.0 | 33.0 |
| 33 | 53.0 | 47.0 |
| 0 | 0.0 | 100.0 |

Table IV

| Evaluation of the transfer of <sup>99m</sup>technetium as <sup>99m</sup>Tc-Saccharate to reduced vs. non-reduced antibody/fragments. | | |
|---|---|---|
| Ab | % Labeling at (1HR) | % Labeling at (3HR) |
| R11D10 IgG | 3.6 | 3.6 |
| R11D10 F(ab')$_2$ | 1.6 | 1.0 |
| R11D10 Fab-DTPA | 4.4 | 3.2 |
| R11D10 Fab' | 100.0 | 100.0 |
| 19-9 IgG | 4.0 | 4.1 |
| 19-9 F(ab)$_2$ | 4.3 | 2.4 |
| 19-9 Fab' | 100.0 | 100.0 |
| 17-1A IgG | 2.7 | 1.5 |
| 17-1A F(ab')$_2$ | 3.0 | 1.4 |
| 17-1A Fab' | 100.0 | 100.0 |

Table V

| Stability of <sup>99m</sup>Tc-labeled 17-1A Fab' and <sup>99m</sup>Tc-labeled R11D10 Fab' in the Absence and Presence of Human Plasma | | | | |
|---|---|---|---|---|
| | % Tc-labeled Ab | | | |
| | 3 hours | | 20 hours | |
| | Absence H. Plasma | Presence H. Plasma | Absence H. Plasma | Presence H. Plasma |
| 17-1A Fab' | 82 | 85 | 93 | 84 |
| R11D10 Fab' | 82 | 83 | 86 | 86 |

Table VI

| Immunoreactivity of Tc-labeled R11D10 Fab' | | | |
|---|---|---|---|
| | % of Binding | | |
| | 0 hours | 3 hours | 20 hours |
| Tc-17-1A Fab' | 1.2 | 2.2 | 3.3 |
| R11D10 Fab' | 81 | 79 | 70 |

## Table VII

Biodistribtuion in % injected dose per gram of Indium labeled R11D10 Fab-DTPA and Technetium-99m labeled R11D10 Fab' at 1,4, and 8 hours post injection in mice.

| Tissue | One Hour In-111- | One Hour 99m Tc- | Four Hours In-111- | Four Hours 99m Tc- | Eight Hours In-111- | Eight Hours 99m Tc- |
|---|---|---|---|---|---|---|
| Blood | 13.62±04.52 | 06.92±00.64 | 04.82±01.00 | 05.11±00.49 | 02.02±00.48 | |
| Spleen | 03.36±01.51 | 03.26±01.53 | 04.51±03.04 | 04.97±01.11 | 02.31±01.17 | |
| Stomach | 02.34±00.83 | 02.07±00.25 | 00.89±00.35 | 01.51±01.04 | 00.31±00.23 | |
| Intestine | 02.74±01.00 | 02.72±00.18 | 01.49±00.04 | 02.53±00.07 | 00.75±00.24 | |
| Kidney | 115.00±32.30 | 36.88±09.38 | 81.10±15.40 | 58.90±05.10 | 57.67±22.61 | |
| Liver | 06.44±01.87 | 08.82±00.91 | 03.68±00.52 | 10.79±00.36 | 02.36±00.95 | |
| Lung | 07.89±03.32 | 05.62±01.60 | 03.60±00.93 | 03.74±00.87 | 01.61±00.57 | |
| Heart | 08.77±04.16 | 03.00±00.28 | 03.00±01.13 | 03.10±00.80 | 01.33±00.05 | |
| Muscle | 01.31±00.17 | 01.53±00.16 | 00.83±00.32 | 01.82±00.5 | 00.42±00.11 | |
| Bone | 00.63±00.24 | ---- | 00.90±00.17 | ---- | 00.92±00.38 | |

--- Not available

A. Example 12: One Vial Method

1. Preparation of the T2G1s Fab' Antibody Fragment

T2G1s $F(ab')_2$ antibody fragment (162 mg) in tris buffer (15.5 ml, 0.05M, pH 8.0) with sodium chloride (0.1M) was treated with DTT (1mM) for 1-2 hours at ambient temperature. The resulting mixture was purified by diafiltration under argon by exchange with 20 volumes of sodium phosphate buffer (0.05M, pH 6.4) containing sodium chloride (0.1M) and EDTA (0.001M) to yield a solution containing T2G1s Fab' (135 mg, concentration 1 mg/mL).

2. Preparation of a Single Vial Kit for Technetium-99m Labeling of T2G1s Antifibrin Antibody Fab' Fragment

To a degassed solution (5 mL) of monopotassium D-glucaric acid (12.5 mg/mL, 0.05M) in potassium phosphate buffer (0.05M, pH 6.4) with EDTA (0.0005M), and sodium chloride (0.16M) was added stannous chloride solution (7.5 uL, 0.1 mg/mL in 1N HCl). To this solution (4.7 mL) was added a solution of murine monoclonal antibody Fab' fragment derived from cell line T2G1s (0.312 mL, 8 mg/mL in 0.05M potassium phosphate buffer pH 6.4 containing 0.1M sodium chloride and 0.001M EDTA) prepared as described in subsection A.1 above. After thorough mixing, portions (1.0 mL) were dispensed into serum vials, lyophilized then sealed with a rubber vial closure.

3. Radiolabeling the Antibody Fab' Fragment with Technetium-99m in a Single Vial Kit

In a one vial procedure sodium ($^{99m}$Tc) pertechnetate (1.0 mL, 20 mCi) was added to the vial of T2G1s Fab' described in subsection A.2 above. The solution was allowed to stand at ambient temperature and the mixture was analyzed at intervals using chromatography on Whatman™ 3MM paper eluting with acetonitrile:water (7:3). In this system, product remained at the origin while ($^{99m}$Tc) pertechnetate and reduced complexed technetium-99m migrated off the origin. Completeness of reaction was determined by the percent of radiolabeled product at the origin. Further dilutions were made, if required, using saline (0.9%). The product of this one vial method was further analyzed as described in Section C below.

B. Comparative Experiments: Two Vial Methods

1. Preparation of a Two Vial Kit for Technetium-99m Labeling of T2G1s Antifibrin Antibody Fab' Fragment

a) Solution Formulation of the Antibody Fragment

A vial was prepared to contain T2G1s Fab' (0.5 mg), prepared substantially as described above in subsection A.1 above, in a buffer solution (1.0 mL) of potassium phosphate (0.05M, pH 6.4), sodium chloride (0.1M) and EDTA (0.001M). The vial was sealed with a rubber vial closure.

b) Lyophilized Formulation of the Antibody Fragment

A vial was prepared to contain T2G1s Fab' (0.5 mg), prepared substantially as described above in subsection A.1 above, in a buffer solution (1.0 mL) of potassium phosphate (0.05M, pH 6.4), sodium chloride (0.05M), lactose (0.05M) and EDTA (0.0005M). The contents of the vial were lyophilized and then sealed with a rubber vial closure.

2. Preparation of the Stannous Composition for the Two Vial Method

Using anaerobic conditions, vials were prepared to contain a solution (1.0 ml) of monopotassium D-glucaric acid (12.5 mg, 0.05 mmol), stannous chloride (150 ug, 0.79 umol) and sodium bicarbonate (16.8 mg, 0.2 mmol, pH 7.6). The contents of vial were lyophilized and then sealed with a rubber vial closure.

3. Radiolabeling the Antibody Fab' Fragment with Technetium-99m in Two Vial Kit

In the two vial procedures sodium ($^{99m}$Tc) pertechnetate (1.0 mL, 20 mCi) was added to the stannous composition described above. After 10 minutes 0.5 mL of this solution was added to the solution and lyophlized formulations of the T2G1s Fab' described in subsections a) and b) above. The product was analyzed as described in Section C.

C. Comparison of Labeled T2G1s Antifibrin Antibody Fab' Fragments

1. Determination of the Immunoreactivity of the Technetium-99m Labeled T2G1s Fab'

Immunoreactivity of the labeled antibody was tested by applying an aliquot of the antibody reaction mixtures to an affinity column (the first seven amino acids of the amino terminus of the beta chain of human fibrin, coupled to CNBr-Sepharose® 6B). The volume of the packed bed was 1 mL. The column was eluted with 10 mL of 1% BSA in 0.01M sodium phosphate, 0.145M NaCl, pH 7.0, followed by elution with 10 mL of 0.1M glycine, pH 2.5. During these elutions, 1 milliliter fractions were collected and counted in a NaI(Tl) well counter. The percent immunoreactivity was computed as:

$$\% \text{ immunoreactivity} = \frac{\text{total net counts eluted by glycine}}{\text{total net counts eluted by both solutions}} \times 100$$

The results are shown in Tables VIII and IX.

2. Comparison of Labeling Rates of the One Vial and Two Vial Kits

Table 1 also shows rates of labeling for the one vial and two vial kits as determined by % protein incorporation according to the paper chromatography technique described in subsection A.3 above. The results show that the one vial kit produces labeled product faster than the two vial kits.

Table VIII

| Labeling Ratio of One Vial and Two Vial Kits | | | | | | | |
|---|---|---|---|---|---|---|---|
| Kit | Storage Temp (°C) | Age When Tested (Days) | % Protein Incorporation | | | | Immunoreactivity (%) |
| | | | 5' | 15' | 30' | 60' | |
| Two Vial Solution | 4° | 7 | NA | 65 | 78 | 87 | 78 |
| Two Vial Lyophilized | 4° | 7 | 61 | 86 | 89 | 93 | 93 |
| One Vial | 4° | 6 | 93 | 94 | 95 | 95 | 98 |

3. Comparison of the Stability of the One Vial and Two Vial Kits

The stability of the one vial and two vial kits were determined by % protein incorporation according to the paper chromatography described in subsection A.3 above at both 4° and 37°. The results are shown in Table 2. The results demonstrate that the one vial kit maintains superior labeling efficiency in the 12-17 day period when stored at 37°.

Table IX

| Stability of One Vial and Two Vial Kits | | | | | | | |
|---|---|---|---|---|---|---|---|
| Kit | Storage Temp (°C) | Age When Tested (Days) | % Protein Incorporation | | | | Immunoreactivity (%) |
| | | | 5' | 15' | 30' | 60' | |
| Two Vial | 4° | 14 | NA | 75 | 84 | 92 | 82 |
| Solution | 37° | 14 | NA | 35 | 45 | 59 | 40 |
| Two Vial | 4° | 17 | 91 | 85 | 94 | 96 | 98 |
| Lyophilized | 37° | 17 | 52 | 80 | 92 | 96 | 98 |
| One Vial | 37° | 12 | 87 | 96 | 97 | 98 | 96 |

Biodistribution in Mice

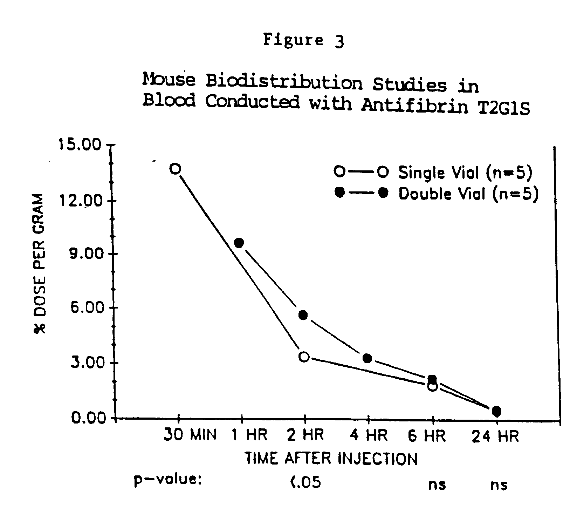

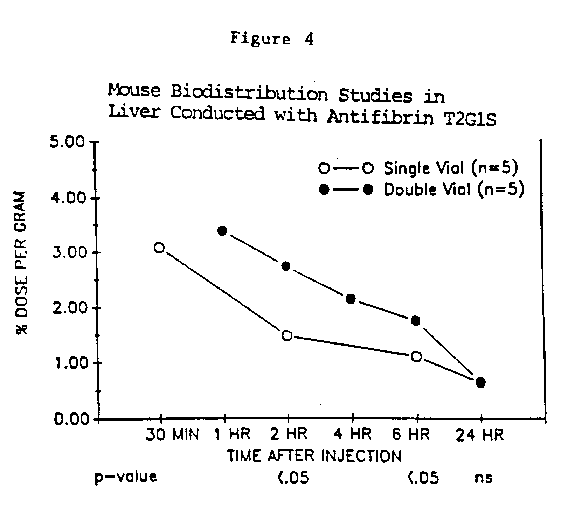

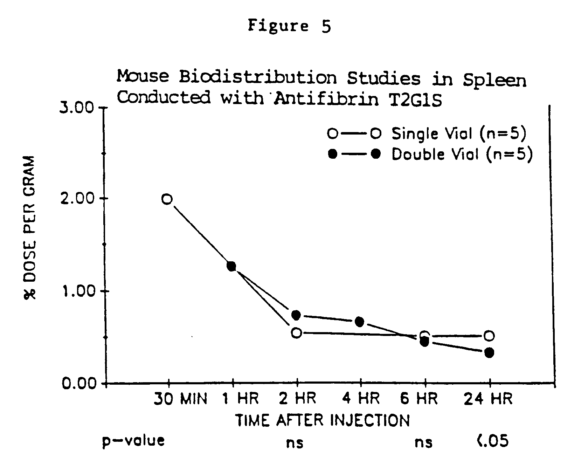

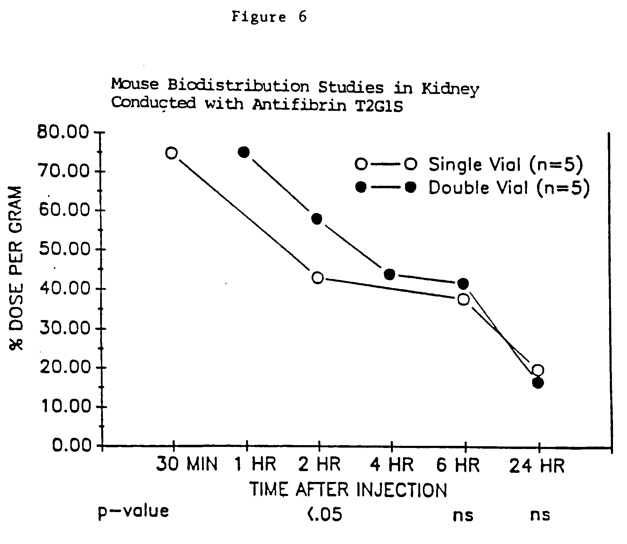

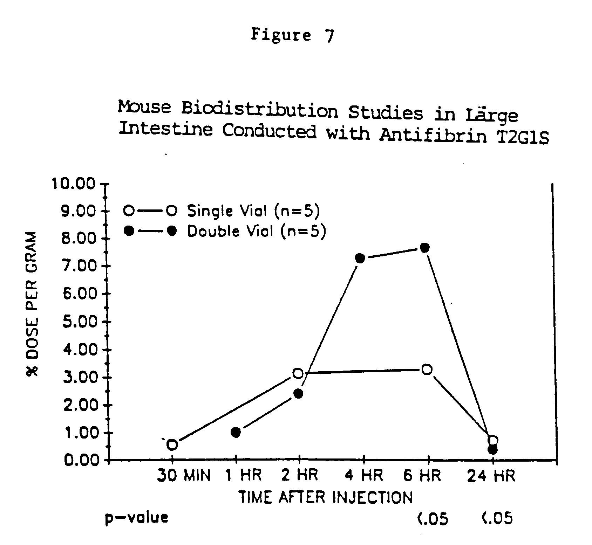

Mouse biodistribution of labeled antibody fragments prepared according to the one vial kit and two vial solution kit described above in Sections A and B was examined by injecting mice I.V. with the labeled fragments and determining the relative amounts of radiolabel accumulated in different tissues. The results are shown in Table X and Figures 3-7. The results show statistically significant differences favoring the one vial kit in every organ system evaluated.

Table X

| ANTIFIBRIN T2G1S MOUSE BIODISTRIBUTION STUDY COMPARISON OF SINGLE VIAL AND DOUBLE VIAL PREPARATIONS MEAN PERCENT DOSE PER GRAM IN FIVE ANIMALS | | | | |
|---|---|---|---|---|
| Organ System | Time After Injection (Hours) | Single Vial (Mean +/- SD) | Double Vial (Mean +/- SD) | p-value |
| Blood | 2.0 | 3.39 ± 0.17 | 5.64 ± 0.53 | <0.05 |
|  | 6.0 | 1.88 ± 0.25 | 2.21 ± 0.28 | ns |
|  | 24.0 | 0.48 ± 0.07 | 0.52 ± 0.08 | ns |
| Heart | 2.0 | 0.90 ± 0.11 | 1.34 ± 0.23 | <0.05 |
|  | 6.0 | 0.56 ± 0.06 | 0.58 ± 0.23 | ns |
|  | 24.0 | 0.45 ± 0.04 | 0.25 ± 0.03 | <0.05 |
| Lungs | 2.0 | 1.36 ± 0.23 | 3.17 ± 0.31 | <0.05 |
|  | 6.0 | 0.88 ± 0.14 | 1.49 ± 0.42 | <0.05 |
|  | 24.0 | 0.41 ± 0.05 | 0.44 ± 0.06 | ns |
| Liver | 2.0 | 1.48 ± 0.17 | 2.73 ± 0.41 | <0.05 |
|  | 6.0 | 1.11 ± 0.16 | 1.75 ± 0.23 | <0.05 |
|  | 24.0 | 0.64 ± 0.03 | 0.65 ± 0.07 | ns |
| Spleen | 2.0 | 0.54 ± 0.15 | 0.73 ± 0.09 | ns |
|  | 6.0 | 0.51 ± 0.12 | 0.45 ± 0.15 | ns |
|  | 24.0 | 0.51 ± 0.09 | 0.33 ± 0.02 | <0.05 |
| Kidneys | 2.0 | 42.98 ± 7.65 | 58.00 ± 6.63 | <0.05 |
|  | 6.0 | 37.70 ± 6.54 | 41.59 ± 9.13 | ns |
|  | 24.0 | 19.85 ± 1.83 | 16.60 ± 2.90 | ns |
| Stomach | 2.0 | 0.34 ± 0.07 | 0.94 ± 0.31 | <0.05 |
|  | 6.0 | 0.85 ± 0.83 | 1.05 ± 0.52 | ns |
|  | 24.0 | 0.35 ± 0.10 | 0.29 ± 0.12 | ns |
| Small Intestine | 2.0 | 1.40 ± 0.44 | 3.63 ± 0.84 | <0.05 |
|  | 6.0 | 0.75 ± 0.22 | 1.38 ± 0.34 | <0.05 |
|  | 24.0 | 0.28 ± 0.04 | 0.24 ± 0.05 | ns |
| Large Intestine | 2.0 | 3.13 ± 0.33 | 2.39 ± 0.89 | ns |
|  | 6.0 | 3.30 ± 1.31 | 7.67 ± 1.96 | <0.05 |
|  | 24.0 | 0.73 ± 0.19 | 0.40 ± 0.11 | <0.05 |
| Muscle | 2.0 | 0.19 ± 0.03 | 0.46 ± 0.20 | <0.05 |
|  | 6.0 | 0.15 ± 0.06 | 0.18 ± 0.03 | ns |
|  | 24.0 | 0.29 ± 0.03 | 0.11 ± 0.03 | <0.05 |
| Gonads | 2.0 | 0.36 ± 0.09 | 1.35 ± 0.39 | <0.05 |
|  | 6.0 | 0.29 ± 0.07 | 0.64 ± 0.14 | <0.05 |
|  | 24.0 | 0.33 ± 0.06 | 0.18 ± 0.03 | <0.05 |

4. Comparison of Plasma Stability of the One Vial and Two Vial Kits

Labeled antibody was prepared as described in the above examples and comparative experiment (solution formulation). The $^{99m}$Tc labeled T2G1s Fab' fragments (100 uL) were added to citrated plasma (50 uL). Table XI compares the plasma stability as determined by % protein incorporation at 37° of the products from the one vial and two vial kits versus control (no plasma added). The results shorn that the plasma stability of the one vial kit is better than the two vial kit.

EP 0 354 923 B1

Table XI

| Plasma Stability of One Vial and Two Vial Kits | | | | | | | | |
|---|---|---|---|---|---|---|---|---|
| Kit | No Plasma Added (Control) % Incorporation | | | | Plasma Added % Incorporation | | | |
| | 15' | 3 hr | 6 hr | 24 hr | 1 hr | 2 hr | 6 hr | 24 hr |
| Two Vial Solution | 92 | 69 | 90 | 78 | 88 | 78 | 73 | 69 |
| One Vial | 88 | 90 | 84 | 89 | 90 | 88 | 86 | 83 |

**Claims**

1. A method of directly labeling a sulfhydryl-containing antibody or antibody fragment with a radiometal selected from the group consisting of technetium-99m, rhenium-186, rhenium-188, rhenium-189 and rhenium-191, comprising the steps of:
   a. forming a mixture of:
   i) the radiometal; and
   ii) a reducing agent and a water-soluble polyhydroxycarboxylic ligand selected from saccharic acid, galactaric acid, arabonic acid and salts thereof, the ligand being capable of complexing the radiometal to form a soluble radiometal-ligand complex;
   b. contacting the mixture with a sulfhydryl-containing antibody or antibody fragment under conditions which permit transfer of the radiometal to the antibody or fragment to form a radiometal-labeled antibody or antibody fragment.

2. A method of Claim 1, wherein the sulfhydryl containing antibody is a reduced IgG.

3. A method of Claim 1, wherein the antibody fragment is a Fab' fragment.

4. A method of Claim 1, wherein Fab' is produced by reducing an $F(ab')_2$ with DTT.

5. A method of claim 1, wherein the reducing agent is a stannous reducing agent.

6. A method of claim 3, wherein the Fab' fragment is derived from anti-myosin, anti-fibrin or anti-platelet antibody.

7. A method of claim 3, wherein the Fab' fragment is derived from an anti-tumor antibody.

8. A method of claim 7, whereing the anti-tumor antibody is an anti-colorectal cancer antibody, anti-ovarian cancer antibody, anti-lung cancer antibody, anti-breast cancer antibody or anti-prostate cancer antibody.

9. A method of claim 3, wherein the Fab' fragment is derived from an antibacterial or anti-macrophage antibody.

10. A method of claim 1 or claim 5, wherein the antibody fragment is a Fab' fragment, the radiometal is technetium-99m in aqueous solution and the water-soluble ligand comprises a saccharic acid or a salt thereof.

11. A method of claim 10 as dependent of claim 5, wherein the mixture of technetium-99m, saccharic acid or salt thereof and stannous reducing agent is formed in a first vial, and the contends of said first vial is thereafter mixed with the contents of a second vial which contains the Fab' fragment in aqueous solution under non-oxidizing conditions.

12. A composition for directly radiolabeling sulfhydryl-containing antibodies or antibody fragments with a radiometal selected from the group consisting of technetium-99m, rhenium-186, rhenium-188, rhenium-189 and rhenium-191, comprising a mixture of the radiometal, a reducing agent and saccharic acid or a salt thereof in an aqueous solution having a pH of from 5 to 9.

18

**13.** A composition of claim 12, wherein the reducing agent is a stannous reducing agent.

**14.** A composition of claim 13 wherein the stannous reducing agent is stannous chloride.

**15.** A kit for radiolabeling a sulfhydryl-containing antibody or antibody fragment with a radiometal selected from the group consisting of technetium-99m, rhenium-186, rhenium-188, rhenium-189 and rhenium-191 comprising:
   a. a vial containing a mixture of a reducing agent and a water soluble polyhydroxycarboxylic ligand selected from saccharic acid, galactaric acid, arabonic acid and salts thereof; and
   b. a vial containing Fab' fragment under non-oxidizing conditions.

**16.** A kit of claim 15, wherein the antibody fragment is a Fab' fragment, the radiometal is technetium-99m and the water soluble ligand is a salt of saccharic acid.

**17.** A kit of claim 15 or claim 16, wherein the reducing agent is a stannous reducing agent.

**18.** A kit of claim, wherein the Fab' fragment is derived from an antimyosin, anti-fibrin or anti-platelet antibody.

**19.** A kit of claim 16, wherein the Fab' fragment is derived from an anti-tumor antibody.

**20.** A kit of claim 19, wherein the anti-tumor antibody is an anti-colorectal cancer antibody, anti-ovarian cancer antibody, anti-breast cancer antibody or anti-prostate cancer antibody.

**21.** A kit of claim 16, wherein the Fab' fragment is derived from an antibacterial or anti-macrophage antibody.

**22.** A method for labeling a sulfhydryl-containing Fab' antibody fragment with technetium-99m comprising:
   a. providing a vial containing a lyophilized mixture of:
      i. the Fab' antibody fragment;
      ii. a stannous reducing agent; and
      iii. D-glucaric acid; and
   b. adding to the vial an aqueous solution of sodium ($^{99m}$TC) pertechnetate.

**23.** A one vial kit for labeling a sulfhydryl containing antibody or antibody fragment with technetium-99m comprising an antibody mixture comprised of a sulfhydryl containing antibody or antibody fragment, a reducing agent and a water-soluble polyhydroxycarboxylic ligand selected from saccharic acid, galactaric acid, arabonic acid and salts thereof, whereby the antibody mixture is contained with technetium-99m in an oxidized state to provide labeled antibody or antibody fragment.

**24.** The one vial kit of claim 23, wherein the antibody mixture is supplied in a lyophilized form.

**25.** A method of claim 1, wherein the conditions which permit transfer of the radiometal are: a temperature of from about 20°C to about 60°C, in an aqueous solution having a pH of from about 5 to about 9, for about 1 hour.

**26.** A method of claim 10, wherein the conditions under which step (b) is performed are: a temperature of from about 20°C to about 60°C, in an aqueous solution having a pH of from about 5 to about 9, for about 1 hour.

**Patentansprüche**

**1.** Verfahren zum direkten Markieren eines Sulfhydryl enthaltenden Antikörpers oder Antikörperfragments mit einem radioaktiven Metall, das ausgewählt ist aus der Gruppe, die aus Technetium-99m, Rhenium-186, Rhenium-188, Rhenium-189 und Rhenium-191 besteht, wobei das Verfahren die folgenden Schritte aufweist:
   a. Bilden eines Gemischs aus:
      i) dem radioaktiven Metall; und

ii) einem Reduktionsmittel und einem wasserlöslichen Polyhydrocarboxyl-Liganden, der ausgewählt ist aus Saccharinsäure, Galactarsäure, Arabonsäure und Salzen davon, wobei der Ligand fähig ist, das radioaktive Metall zu komplexieren, um einen löslichen Radiometall-Ligand-Komplex zu bilden;

b. In-Kontakt-Bringen des Gemischs mit einem Sulfhydryl enthaltenden Antikörper oder Antikörperfragment unter Bedingungen, die die Übertragung des Radiometalls auf den Antikörper oder das Fragment erlauben, um einen Radiometall-markierten Antikörper oder ein solches Antikörperfragment zu bilden.

2. Verfahren nach Anspruch 1, wobei der Sulfhydryl enthaltende Antikörper ein reduziertes IgG ist.

3. Verfahren nach Anspruch 1, wobei das Antikörperfragment ein Fab'-Fragment ist.

4. Verfahren nach Anspruch 1, wobei Fab' durch Reduktion eines $F(ab')_2$ mit DTT erzeugt wird.

5. Verfahren nach Anspruch 1, wobei das Reduktionsmittel ein Zinn(II)-Reduktionsmittel ist.

6. Verfahren nach Anspruch 3, wobei das Fab'-Fragment von Antimyosin-, Antifibrin- oder Antithrombozyt-Antikörper abgeleitet ist.

7. Verfahren nach Anspruch 3, wobei das Fab'-Fragment von einem Antitumor-Antikörper abgeleitet ist.

8. Verfahren nach Anspruch 7, wobei der Antitumor-Antikörper ein Anti-Kolorektalkarzinom-Antikörper, Anti-Ovarialkarzinom-Antikörper, Anti-Lungenkarzinom-Antikörper, Anti-Mammakarzinom-Antikörper oder Anti-Prostatakarzinom-Antikörper ist.

9. Verfahren nach Anspruch 3, wobei das Fab'-Fragment von einem bakterienhemmenden Antikörper oder Anti-Makrophagen-Antikörper abgeleitet ist.

10. Verfahren nach Anspruch 1 oder Anspruch 5, wobei das Antikörperfragment ein Fab'-Fragment ist, das radioaktive Metall Technetium-99m in wäßriger Lösung ist und der wasserlösliche Ligand eine Saccharinsäure oder ein Salz davon aufweist.

11. Verfahren nach Anspruch 10 in Abhängigkeit von Anspruch 5, wobei das Gemisch aus Technetium-99m, Saccharinsäure oder Salz davon und Zinn(II)-Reduktionsmittel in einer ersten Phiole gebildet wird und der Inhalt der ersten Phiole danach mit dem Inhalt einer zweiten Phiole, die das Fab'-Fragment in wäßriger Lösung unter nichtoxidierenden Bedingungen enthält, vermischt wird.

12. Zusammensetzung zum direkten Radiomarkieren von Sulfhydryl enthaltenden Antikörpern oder Antikörper-Fragmenten mit einem radioaktiven Metall, das ausgewählt ist aus der Gruppe, die aus Technetium-99m, Rhenium-186, Rhenium-188, Rhenium-189 und Rhenium-191 besteht, wobei die Zusammensetzung ein Gemisch aus dem radioaktiven Metall, einem Reduktionsmittel und Saccharinsäure oder einem Salz davon in einer wäßrigen Lösung mit einem pH von 5-9 aufweist.

13. Zusammensetzung nach Anspruch 12, wobei das Reduktionsmittel ein Zinn(II)-Reduktionsmittel ist.

14. Zusammensetzung nach Anspruch 13, wobei das Zinn(II)-Reduktionsmittel Zinn(II)-chlorid ist.

15. Set zum Radiomarkieren eines Sulfhydryl enthaltenden Antikörpers oder Antikörper-Fragments mit einem radioaktiven Metall, das ausgewählt ist aus der Gruppe, die aus Technetium-99m, Rhenium-186, Rhenium-188, Rhenium-189 und Rhenium-191 besteht, wobei das Set folgendes aufweist:
a. eine Phiole, die ein Gemisch aus einem Reduktionsmittel und einem wasserlöslichen Polyhydroxycarboxyl-Liganden, der aus Saccharinsäure, Galactarsäure und Arabonsäure und Salzen davon ausgewählt ist, enthält; und
b. eine Phiole, die Fab'-Fragment unter nichtoxidierenden Bedingungen enthält.

16. Set nach Anspruch 15, wobei das Antikörper-Fragment ein Fab'-Fragment, das Radiometall Technetium-99m und der wasserlösliche Ligand ein Salz von Saccharinsäure ist.

20

**17.** Set nach Anspruch 15 oder Anspruch 16, wobei das Reduktionsmittel ein Zinn(II)-Reduktionsmittel ist.

**18.** Set nach Anspruch 16, wobei das Fab'-Fragment von einem Antimyosin-, Antifibrin- oder Antithrombozyt-Antikörper abgeleitet ist.

**19.** Set nach Anspruch 16, wobei das Fab'-Fragment von einem Antitumor-Antikörper abgeleitet ist.

**20.** Set nach Anspruch 19, wobei der Antitumor-Antikörper ein Anti-Kolorektalkarzinom-Antikörper, Anti-Ovarialkarzinom-Antikörper, Anti-Mammakarzinom-Antikörper oder Anti-Prostatakarzinom-Antikörper ist.

**21.** Set nach Anspruch 16, wobei das Fab'-Fragment von einem bakterienhemmenden Antikörper oder einem Anti-Makrophagen-Antikörper abgeleitet ist.

**22.** Verfahren zum Markieren eines Sulfhydryl enthaltenden Fab'-Antikörper-Fragments mit Technetium-99m, wobei das Verfahren folgende Schritte aufweist:
   a. Bereitstellen einer Phiole, die ein lyophilisiertes Gemisch aus folgendem enthält:
      i. dem Fab'-Antikörper-Fragment;
      ii. einem Zinn(II)-Reduktionsmittel; und
      iii. D-Glucarsäure; und
   b. Zufügen einer wäßrigen Lösung von Natrium($^{99m}$TC)-pertechnetat zu der Phiole.

**23.** Ein-Phiolen-Set zum Markieren eines Sulfhydryl enthaltenden Antikörpers oder Antikörper-Fragments mit Technetium-99m, wobei das Set ein Antikörpergemisch aus einem Sulfhydryl enthaltenden Antikörper oder Antikörper-Fragment, ein Reduktionsmittel und einen wasserlöslichen Polyhydroxycarboxyl-Liganden, der ausgewählt ist aus Saccharinsäure, Galactarsäure, Arabonsäure und Salzen davon, aufweist, wobei das Antikörper-Gemisch mit Technetium-99m in einem oxidierten Zustand enthalten ist, um markierten Antikörper oder markiertes Antikörper-Fragment bereitzustellen.

**24.** Ein-Phiolen-Set nach Anspruch 23, wobei das Antikörper-Gemisch in einer lyophilisierten Form bereitgestellt wird.

**25.** Verfahren nach Anspruch 1, wobei die Bedingungen, die die Überführung des radioaktiven Metalls erlauben, folgende sind: eine Temperatur von ca. 20 °C bis ca. 60 °C, in einer wäßrigen Lösung mit einem pH von ca. 5 bis ca. 9, für ca. 1 h.

**26.** Verfahren nach Anspruch 10, wobei die Bedingungen, unter denen Schritt (b) durchgeführt wird, folgende sind: eine Temperatur von ca. 20 °C bis ca. 60 °C, in einer wäßrigen Lösung mit einem pH von ca. 5 bis ca. 9, für ca. 1 h.

**Revendications**

**1.** Procédé de marquage direct d'un anticorps ou fragment d'anticorps contenant le radical sulfhydryle par un métal radioactif choisi dans le groupe formé par le technétium-99m, le rhénium-186, le rhénium-188, le rhénium-189 et le rhénium-191, comprenant les étapes suivantes :
   a. former un mélange de :
      i) le métal radioactif ; et
      ii) un agent réducteur et un ligand polyhydroxycarboxylique hydrosoluble choisi parmi l'acide saccharique, l'acide galactarique, l'acide arabonique et leurs sels, le ligand étant capable de complexer le métal radioactif pour former un complexe soluble métal radioactif-ligand ;
   b. mettre en contact le mélange avec un anticorps ou fragment d'anticorps contenant le radical sulfhydryle dans des conditions permettant le transfert du métal radioactif à l'anticorps ou au fragment pour former un anticorps ou fragment d'anticorps marqué par le métal radioactif.

**2.** Procédé de la revendication 1, dans lequel l'anticorps contenant le radical sulfhydryle est une IgG réduite.

**3.** Procédé de la revendication 1, dans lequel le fragment d'anticorps est un fragment Fab'.

4. Procédé de la revendication 1, dans lequel Fab' est produit par réduction d'un F(ab')$_2$ avec DTT.

5. Procédé de la revendication 1, dans lequel l'agent réducteur est un agent réducteur stanneux.

6. Procédé de la revendication 3, dans lequel le fragment Fab' est dérivé d'un anticorps antimyosine, antifibrine ou antiplaquettaire.

7. Procédé de la revendication 3, dans lequel le fragment Fab' est dérivé d'un anticorps antitumoral.

8. Procédé de la revendication 7, dans lequel l'anticorps antitumoral est un anticorps anti-cancer rectocolique, un anticorps anti-cancer ovarien, un anticorps anti-cancer du poumon, un anticorps anti-cancer du sein ou un anticorps anti-cancer de la prostate.

9. Procédé de la revendication 3, dans lequel le fragment Fab' est dérivé d'un anticorps antibactérien ou antimacrophages.

10. Procédé de la revendication 1 ou la revendication 5, dans lequel le fragment d'anticorps est un fragment Fab', le métal radioactif est le technétium-99m en solution aqueuse et le ligand hydrosoluble comprend un acide saccharique ou un sel de celui-ci.

11. Procédé de la revendication 10 sous la dépendance de la revendication 5, dans lequel le mélange de technétium-99m, d'acide saccharique ou de son sel et d'agent réducteur stanneux est formé dans un premier flacon, et le contenu de ce premier flacon est ensuite mélangé avec le contenu d'un second flacon qui contient le fragment Fab' en solution aqueuse dans des conditions non oxydantes.

12. Composition pour le radiomarquage direct d'anticorps ou fragments d'anticorps contenant le radical sulfhydryle par un métal radioactif choisi dans le groupe formé par le technétium-99m, le rhénium-186, le rhénium-188, le rhénium-189 et le rhénium-191, comprenant un mélange du métal radioactif, d'un agent réducteur et d'acide saccharique ou d'un sel de celui-ci dans une solution aqueuse ayant un pH de 5 à 9.

13. Composition de la revendication 12, dans laquelle l'agent réducteur est un agent réducteur stanneux.

14. Composition de la revendication 13, dans laquelle l'agent réducteur stanneux est le chlorure stanneux.

15. Nécessaire pour le radiomarquage d'un anticorps ou fragment d'anticorps contenant le radical sulfhydryle par un métal radioactif choisi dans le groupe formé par le technétium-99m, le rhénium-186, le rhénium-188, le rhénium-189 et le rhénium-191, comprenant :
   a. un flacon contenant un mélange d'un agent réducteur et d'un ligand polyhydroxycarboxylique hydrosoluble choisi parmi l'acide saccharique, l'acide galactarique, l'acide arabonique et leurs sels ; et
   b. un flacon contenant un fragment Fab' dans des conditions non oxydantes.

16. Nécessaire de la revendication 15, dans lequel le fragment d'anticorps est un fragment Fab', le métal radioactif est le technétium-99m et le ligand hydrosoluble est un sel d'acide saccharique.

17. Nécessaire de la revendication 15 ou la revendication 16, dans lequel l'agent réducteur est un agent réducteur stanneux.

18. Nécessaire de la revendication 16, dans lequel le fragment Fab' est dérivé d'un anticorps antimyosine, antifibrine ou antiplaquettaire.

19. Nécessaire de la revendication 16, dans lequel le fragment Fab' est dérivé d'un anticorps antitumoral.

20. Nécessaire de la revendication 19, dans lequel l'anticorps antitumoral est un anticorps anti-cancer rectocolique, un anticorps anti-cancer ovarien, un anticorps anti-cancer du sein ou un anticorps anti-cancer de la prostate.

**21.** Nécessaire de la revendication 16, dans lequel le fragment Fab' est dérivé d'un anticorps antibactérien ou antimacrophages.

**22.** Procédé de marquage d'un fragment d'anticorps Fab' contenant le radical sulfhydryle par le technétium-99m, consistant à
   a. obtenir un flacon contenant un mélange lyophilisé de :
      i. le fragment d'anticorps Fab' ;
      ii. un agent réducteur stanneux ; et
      iii. acide D-glucarique ; et
   b. ajouter au flacon une solution aqueuse de ($^{99m}$Tc)-pertechnétate de sodium.

**23.** Nécessaire à flacon unique pour le marquage d'un anticorps ou fragment d'anticorps contenant le radical sulfhydryle par le technétium-99m, comprenant un mélange d'anticorps constitué d'un anticorps ou fragment d'anticorps contenant le radical sulfhydryle, d'un agent réducteur et d'un ligand polyhydroxycarboxylique hydrosoluble choisi parmi l'acide saccharique, l'acide galactarique, l'acide arabonique et leurs sels, au moyen duquel le mélange d'anticorps est mis en contact avec du technétium-99m en un état oxydé pour produire un anticorps ou fragment d'anticorps marqué.

**24.** Nécessaire à flacon unique de la revendication 23, dans lequel le mélange d'anticorps est fourni sous forme lyophilisée.

**25.** Procédé de la revendication 1, dans lequel les conditions qui permettent le transfert du métal radioactif sont : une température d'environ 20°C à environ 60°C, dans une solution aqueuse ayant un pH d'environ 5 à environ 9, pendant environ 1 heure.

**26.** Procédé de la revendication 10, dans lequel les conditions dans lesquelles est exécutée l'étape (b) sont : une température d'environ 20°C à environ 60°C, dans une solution aqueuse ayant un pH d'environ 5 à environ 9, pendant environ 1 heure.

FIG.1

FIG.2

Figure 3

Mouse Biodistribution Studies in
Blood Conducted with Antifibrin T2G1S

Figure 4

Mouse Biodistribution Studies in
Liver Conducted with Antifibrin T2G1S

Figure 5

Mouse Biodistribution Studies in Spleen
Conducted with Antifibrin T2G1S

Figure 6

Mouse Biodistribution Studies in Kidney
Conducted with Antifibrin T2G1S

Figure 7

Mouse Biodistribution Studies in Large
Intestine Conducted with Antifibrin T2G1S